# EUROPEAN PATENT APPLICATION

(11) **EP 3 698 838 A1**
(43) Date of publication of application: **26.08.2020**
(21) Application number: 19158351.7
(22) Date of filing: 20.02.2019
(51) Int. Cl.: A61M 16/12, B01F 3/02, A61M 16/00

(54) **GAS MIXER FOR PROVING A GAS MIXTURE USEABLE IN HOSPITALS**

(71) Applicant: L'AIR LIQUIDE, SOCIETE ANONYME POUR L'ETUDE ET L'EXPLOITATION DES PROCEDES GEORGES CLAUDE, 75007 Paris (FR)
(72) Inventor: BOULANGER, Thierry, Newark, DE Delaware DE-19702 (US)
(74) Representative: Pittis, Olivier

(57) **Abstract**

The invention concerns a gas mixer (1) comprising a first mixing vessel (42) and a second mixing vessel (52); a first line (22) for providing a first gas and a second line (32) for providing a second gas, the first and second lines (22,32) being in fluid communication with the first mixing vessel (42) and with the second mixing vessel (52) for proving said first and second gases to said first and second mixing vessels (42, 52); and a third line (12) for providing a third gas, said third line (12) being in fluid communication with the second mixing vessel (52). Mechanical ventilation system comprising gas mixer (1) according the invention in fluid communication with a mechanical ventilator (9) for providing gas or gas mixtures to said mechanical ventilator (9). Ventilation assembly comprising a gas mixer (1) according to the present invention in fluid communication with a blender device (10) for providing gas or gas mixtures to said blender device (10), especially an argon-containing gas mixture. Such a gas mixer (1) is useable in hospitals or the like for providing gas mixtures used for treating respiratory deficiencies or diseases affecting different populations of patients.

## Description

The present invention relates to a gas mixing device or gas mixer that can be used in hospitals or similar healthcare facilities for operating precise deliveries of various gas mixtures, especially when fluidly connected to a medical ventilator or to a gas blender device.

Gas mixtures are commonly used for treating respiratory deficiencies or diseases affecting different populations of patients, or for other purposes, such as alleviating pain, neuroprotection...

Medical gas mixtures typically contain 2 or 3 compounds, such as oxygen, inert gases and/or therapeutically-effective gases. For instance He/O₂, Xe/O₂, N₂O/O₂, NO/N₂/O₂... are known mixtures used in hospitals or the like.

Several technical possibilities exist for generating ternary gas mixtures.

For instance, US-A-2009/0205661 discloses a gas mixer that is fluidly connected to the air inlet of a medical ventilator. This gas mixer comprises three gas lines with flow controllers for providing the gas compounds and generating the desired gas mixture in a pressurized vessel, before delivering it to the ventilator. However, such a gas mixer is not ideal as the gaseous mixture delivered by the mixer depends on the O₂ concentration set on the ventilator itself. Hence, when the O₂ concentration changes, the gaseous mixture has to vary accordingly and the pressurized vessel has to be flushed, which means that gases are wasted. Further, a precise mixture cannot always be realized as it closely depends on the ventilator itself, especially on the precision of its internal mixer, which may vary from one ventilator to another.

In other words, making a ternary gaseous mixture that keeps the concentration of a therapeutic gas within tight tolerances, regardless of the conditions of operation, e.g. with or without cooperation with a mechanical ventilator, and for variable oxygen concentrations, is not obvious.

Hence, a problem to be solved is to provide an improved gas mixer that can be used for providing ternary gas mixtures containing a therapeutically-effective gas(es) at a concentration within tight tolerances, including when the oxygen concentration varies, and that can be further fluidly connected to a medical ventilator for providing said ternary gas mixtures to said medical ventilator.

A solution according to the present invention is a gas mixer, also called "gas mixing device", "gas blender" or the like, comprising :
- a first mixing vessel and a second mixing vessel,
- a first line for providing a first gas and a second line for providing a second gas, the first and second lines being in fluid communication with the first mixing vessel and with the second mixing vessel, respectively, for proving said first and second gases to said first and second mixing vessels, and
- a third line for providing a third gas, said third line being in fluid communication with the second mixing vessel, i.e. said third line is only fluidly connected to the second mixing vessel, but not to the first one.

Depending on the embodiment, the gas mixer according to the present invention can comprise one or several of the following features :
- the first line is fluidly connected to the second mixing vessel by a first additional line.
- the second line is fluidly connected to the second mixing vessel by a second additional line.
- the first line, the second line and the third line comprise gas conducts or the like.
- the first additional line comprises a flow control device.
- the flow control device is a solenoid valve.
- the second additional line further comprises a first flow sensor.
- the second common line comprises a second flow sensor.
- the first and second lines are in fluid communication with the first mixing vessel via a first common line section.
- the first, second and third lines are in fluid communication with the second mixing vessel via a second common line section.
- the first common line section and the second common line section comprise gas conducts or the like.
- the first line comprises a first solenoid valve and/or a first pressure regulator, preferably the first pressure regulator is arranged downstream of the first solenoid valve.
- the second line comprises a second solenoid valve and/or a second pressure regulator, preferably the second pressure regulator is arranged downstream of the second solenoid valve.
- the third line comprises a third solenoid valve and/or a third pressure regulator, preferably the third pressure regulator is arranged downstream of the third solenoid valve.
- the first line comprises a first calibrated orifice, preferably arranged downstream of the first pressure regulator.
- the second line comprises a second calibrated orifice, preferably arranged downstream of the second pressure regulator.
- the third line comprises a third calibrated orifice, preferably arranged downstream of the third pressure regulator.
- a first oxygen sensor is arranged in first common line section.
- a second oxygen sensor is arranged in second common line section.
- the first line is fed by a first gas source containing oxygen, i.e. an oxygen source.
- the second line is fed by a second gas source containing a therapeutically-effective gas, preferably argon.
- the third line is fed by a third gas source containing air, i.e. an air source (e.g. 78%N₂/22%O₂ mixture; vol%).
- the first gas source comprises an oxygen-containing cylinder, an oxygen line or the like.
- the second gas source comprises an argon-containing cylinder or the like.
- the third gas source comprises an air- or N₂/O₂ mixture -containing cylinder, or an air or N₂/O₂ mixture line, or the like.
- the gas mixer further comprises a control unit, i.e. pilot means or device.
- the gas mixer further comprises a control unit comprising a microprocessor.
- the control unit comprises a electronic board, preferably comprising a microprocessor, such as a microcontroller.
- the solenoid valve of the first additional line is controlled by the control unit.
- the control unit is electrically connected to the first flow sensor of the second additional line.
- the control unit is electrically connected to the second flow sensor of the second common line.
- the control unit is electrically connected to the first oxygen sensor of the first common line section.
- the control unit is electrically connected to the second oxygen sensor of the second common line.
- the gas mixer further comprises a command unit operable by a user, also called man-machine interface, such as a keyboard and/or a touch screen or the like, for setting the desired concentrations of the different gases in the mixture(s) to be realized in the vessels.
- the control unit is electrically connected to the command unit.
- the gas mixer further comprises a housing.
- the first, second and third lines, and the first and second vessels are arranged in the housing.
- the gas mixer further comprises a power supply, such as battery(ies), or electrical cord and socket, for providing electrical current/power to the mixer elements in need thereof, such as the control unit.
- the control unit functions to operate (i.e. pilots) the first solenoid valve and the first pressure regulator of the first line.
- the control unit functions to operate (i.e. pilots)the second solenoid valve and the second pressure regulator of the second line.
- the control unit functions to operate (i.e. pilots)the third solenoid valve and the third pressure regulator of the third line.

The present invention further concerns a mechanical ventilation system comprising a gas mixer according to the present invention in fluid communication with a mechanical ventilator for providing gas or gas mixtures to said mechanical ventilator, especially an argon-containing gas mixture.

The present invention further concerns a ventilation assembly comprising a gas mixer according to the present invention in fluid communication with a blender device for providing gas or gas mixtures to said blender device, especially an argon-containing gas mixture.

A method for manufacturing a ternary gas mixture comprising :
a) providing a gas mixer according to the present invention,
b) providing a first gas, a second gas and a third gas,
c) mixing the first gas, second gas and third gas into the gas mixer according to the present invention, and
d) recovering at least a ternary gas mixture comprising said first, second and third gases.

Depending on the embodiment, the method according to the present invention can comprise one or several of the following features :
- the ternary gas mixture is provided to a medical ventilator or to a gas blender device and preferably further administrated to a patient (i.e. adult, teenager, infant, toddler or neonate) in need thereof.
- the first gas is oxygen.
- the second gas comprises a therapeutically-effective gas, preferably argon.
- the third gas is air (e.g. 78%N₂/22%O₂ mixture; vol%).

The present invention will be explained in more details in the following illustrative description of an embodiment of a gas mixing device or gas mixer according to the present invention, which is made in references to the accompanying drawings among them :
- Figure 1 shows an embodiment of the architecture of a gas mixer according to the present invention,
- Figure 2 shows a gas mixer according to the present invention fluidly connected to a mechanical ventilator, and
- Figure 3 shows the gas mixer according to the present invention fluidly connected to a gas blender device.

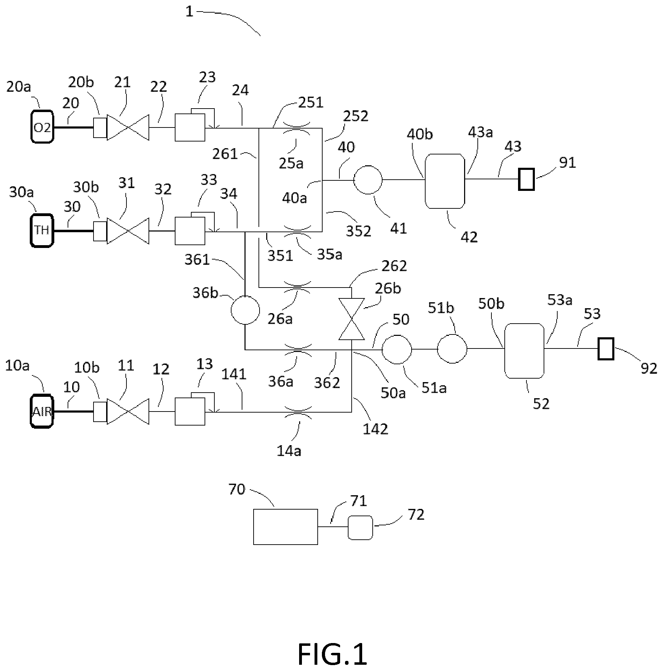

In Figure 1, a first embodiment of a gas mixer 1 according to the present invention is shown. It comprises a first line 22, a second line 32 and a third line 12, such as gas conducts, passages or the like, arranged in parallel and comprising a first inlet port 20b, a second inlet port 30b and a third inlet port 10b, respectively.

The first port 20b is fluidly connected, via a first inlet section 20, to a first source 20a of a first gas, namely a medical O₂ source 20a (02 in Fig. 1), for instance an oxygen delivery plug (i.e. gas-connector) also fed by the gas network of the hospital, or an oxygen-containing cylinder or reservoir.

The second port 30b is fluidly connected, via a second inlet section 30, to a second source 30a of a second gas, namely a therapeutically-effective gas (TH in Fig. 1) source 30a, preferably a gas cylinder, a gas reservoir or the like. The therapeutically-effective gas is argon, N₂O or xenon. Preferably, the therapeutically-effective gas is argon.

The third inlet port 10b is fluidly connected, via a third inlet section 10, to a third source 10a of a third gas, namely a medicinal air source (AIR in Fig. 1), for instance an air delivery plug (i.e. gas-connector), generally arranged on a wall, and fed by the gas network of a hospital, or an air-containing cylinder or reservoir.

According to the present invention, the first 22 and second 32 lines are fluidly connected to two mixing vessels, namely a first mixing vessel 42 and a second mixing vessel 52, for proving said first and second gases to said first and second mixing vessels 42, 52, whereas the third line 12 is in fluid communication only with the second mixing vessel 52, i.e. it is not fluidly connected to the first mixing vessel 42, for providing the third gas, i.e. medicinal air only to the second mixing vessel 52 (i.e. not to the first one 42).

The gas mixer 1 according to the present invention further comprises different electronic elements, such as pressure regulators, solenoid valves and sensors that are controlled by a control unit 70. Control unit 70 comprises a microprocessor, preferably a microcontroller, for instance it comprises a electronic board with microprocessor(s), memory(ies)....

Control unit 70 further provides electric power to the electronic elements and is able to read and to process the measurements provided by the sensors, typically signals. Control unit 70 also embeds/memorizes conversion lookup tables or the like, for determining a flow measurement based on the nature of the gas being measured, for instance medicinal air, O₂ and argon as the therapeutic gas.

Control unit 70 is further in electrical communication, via electric line 71, to a command unit 72 operable by a user, such as a keyboard, a touch screen or the like, for setting the desired concentrations of the different gases in the mixture(s) to be realized in vessels 42 and 52.

Third line 12 comprises, arranged in series, downstream of the third inlet port 10b (i.e. air port), a third solenoid valve 11 and a third pressure regulator 13.

Third solenoid valve 11 is preferably a 2:2 normally closed valve that prevents gas losses in case the gas mixer 1 is not in operation, and shuts down the gas supply in case of failure of the gas mixer 1. In normal operation, control unit 70 opens the third solenoid valve 11.

The third pressure regulator 13 is provided for reducing the pressure of the third gas, e.g. air, delivered by the third gas source 10a. The level of the desired lower pressure depends on the settings of the third pressure regulator 13. For instance, if the pressure of the third gas is of about 3.5 bars abs, the pressure level of the third pressure regulator 13 can be set to a lower pressure of 2.5 bars abs (of course, it could be higher or lower) so that the third gas exhibits said lower pressure downstream of the third pressure regulator 13, in downstream line 141. For instance, the third pressure regulator 13 is an electronic pressure regulator, such as the SPV series from Proportion-Air. The downstream pressure is set by control unit 70 and the third pressure regulator 13 keeps this downstream pressure steady without drift overtime, for instance +/- %0.25 with respect to the pressure value.

Third pressure regulator 13 can also indicate to control unit 70 that no or insufficient pressure exists in third line 12. This happens for instance when the third source 10a is not connected to the third inlet port 10b.

The gas traversing the third pressure regulator 13 is recovered in a downstream section 141 of third line 12 that comprises a calibrated orifice 14a, preferably made by precision machining, so that its diameter has very tight tolerances. The gas passing through calibrated orifice 14a, is recovered in another section 142 of third line 12. Calibrated orifice 14a is the main restrictive element of sections 141, 142 so that the flow resistance generated in downstream sections 141,142 is negligible in comparison to the flow resistance generated by calibrated orifice 14a.

Similarly, the first gas line 22 and second line 32 comprise, arranged in series, downstream of the first and second inlet ports 20b, 30b (i.e. the O₂ port and the therapeutically-effective gas port), first and second solenoid valves 21, 31 and a first and second pressure regulators 23, 33. Further, here again, downstream sections 24, 34 of the first and second lines 22, 32, respectively, comprise each a calibrated orifice 25a, 35a. Said calibrated orifices 25a, 35a are arranged in downstream sections 24, 34 of first and second lines 22, 32 thereby dividing said downstream sections 24, 34 in sub-portions 251, 252; 351, 352, respectively, as shown in Figure 1. Those elements of first and second lines 22, 32 are the same or similar, and run or are operated the same way as the corresponding ones that are arranged in the third line 12.

As one can see in Fig. 1, the first 22 and second 32 lines are fluidly connected, at a first location 40a, to the first mixing vessel 42 by means of a first common line section 40, such a pipe, conduct or the like.

Similarly, the first, second and third lines 22, 32,12 are in fluid communication with the second mixing vessel 52 via a second common line section 50, such a pipe, conduct or the like.

First and second lines 22, 32 are fluidly connected to the second common line section 50 by, respectively, a first additional line 261 and a second additional line 361. In other words, said first and a second additional lines 261, 361 are branched lines fluidly linking said first 22 and second 32 lines to the second common line section 50 for providing gases to the second mixing vessel 52 and mixing them therein.

The first additional line 261 further comprises a solenoid valve 26b that is a normally open 2:2 valve, which can be closed by control unit 70 if need be. In other words, solenoid valve 26b is controlled by the control unit 70.

The first additional line 261 is terminated by a first end portion 262, whereas the second additional line 361 is terminated by a second end portion 362, said first end portion 262 and second end portion 362 being fluidly connected to downstream section 142 of third line 12 at a second location 50a.

The second additional line 361 further comprises a flow sensor 36b which measures the flow traveling into said second additional line 361. Flow sensor 36b is preferably configured by a lookup table relating to the second gas, such as argon.

The mixer 1 of the present invention is configured for delivering various gas mixtures, especially ternary mixtures, obtained by mixing gases provided by the first, second and third gas sources 20a, 30a, 10a.

For instance, when the mixer 1 is switched on by a user, upon actuation of the solenoid valves 21, 31, 11 by control unit 70, the first, second and third pressure regulators 13, 23, 33 can inform the control unit 70 of the presence of gas sources fluidly connected to the first, second and third ports 20b, 30b, 10b. The solenoid valves 11, 21 and 31 are open when the control unit 70 determined the presence of the gas sources, i.e. when the gases to be mixed are available and provided to the first, second and third ports 20b, 30b, 10b. First and second pressure regulators 23, 33 are set at the same pressure, for instance 2.5 bars, by the control unit 70 so that the gas pressure is the same in all the arranged downstream of said regulators 23, 33, namely in lines 24, 34, 251, 261, 351, 361, as well in calibrated orifices 25a, 35a.

The downstream portions 252, 352 of sub-portions 251, 351 are branched, i.e. connected, at first location 40a, and contain gas at a same pressure. This means that the pressure drops across orifices 25a, 35a are the same. For instance, orifice 25a has an internal diameter of between 0.1 and 3 mm, preferably of about 1 mm, whereas orifice 35a has an inner diameter about 10% larger than the one of orifice 25a. Therefore, the flow spreading into first common line 40 is made of a mixture equivalent to 50% (v/v) Ar and 50% (v/v) O₂. This mixture enters into first mixing vessel 42 that is located at the terminal end 40b of first common line 40.

The first mixing vessel 42 has an inner volume typically comprised between 0.1 and 5 L, for example of about 0.5 L, where the gas mixture is stored at a pressure not exceeding the pressure set on pressure regulators 23, 33, e.g. 2.5 bars. First vessel 42 constitutes a mixing chamber for the gases as well as a buffer.

For security reason, an oxygen sensor 41 is arranged in first common line 40, upstream of vessel 42. Oxygen sensor 41 is for example a zirconia based sensor, such as the one referenced KGZ-10 commercialized by the Honeywell, which is designed for operating at pressures up to 3 bar abs. Such an oxygen sensor is linear while operating, meaning that its output is linearly dependent on the oxygen concentration (i.e. from 0 to 100%).

Control unit 70 may rely on the output of oxygen sensor 41 to determine that the gas mixture is correct, e.g. that it contains 50% (v/v) O₂, the balance being Ar.

If the measured concentration far differs from 50% (v/v), control unit 70 takes appropriate actions and shut off second solenoid valve 31 to stop the delivery of the therapeutic gas, i.e. argon. Therefore, only O₂ from first line 22 is able to travel into first common line section 40 to fill the first mixing vessel 42.

The gas mixture obtained in the first mixing vessel 42 is recovered by a first exit line 43 and delivered by first outlet port 91 located at the end of the first exit line 43.

In the same way, assuming that the third pressure regulator 13 is set at the same pressure than first and second pressure regulators 23, 33, a same gaseous pressure is obtained in downstream line 141 as in downstream portions 261, 361. Further, the downstream line 142 and downstream portions 262, 362, that are branched at second location 50a, also contain a same pressure of gas, i.e. downstream of the calibrated orifices 14a, 26a, 36a, and in the second common line 50.

Calibrated orifices 14a, 26a, 36a are designed so that, for a same pressure drop, and assuming a given volumetric flow (Qar) through calibrated orifice 36a arranged in the second additional line 361, the flow through the first calibrated orifice 26a (i.e. O₂) is of about 25% of Qar, whereas the flow through the third calibrated orifice 14a (i.e. air) is of about 75% of Qar. This means that the gas traveling into second common line 50 is a ternary mixture composed of 50% (v/v) Ar, 29% (v/v) N₂ (i.e. air) and 21 % (v/v) O₂.

Second pressure vessel 52 is similar to and works as the first pressure vessel 42, i.e. same volume and gas pressure (e.g. 2.5 bar abs), for mixing the gases and storing them (i.e. buffer). Similarly, another oxygen sensor 51 a is located in second common line 50, preferably upstream of vessel 52 for ensuring that the amount of O₂ is of at least 21 % (v/v).

Further, flow sensors 36b measures the therapeutic gas flow provided by second additional line 361, whereas an additional flow sensor 51b of the second common line 50, measures the total flow of gas circulating in said second common line 50.

Control unit 70 receives the measurement signals from said flow sensors 36b, 51 b and, if need be, applies appropriate lookup tables, to determine that the flow measured by flow sensor 51b is twice the flow measured by flow sensor 36b, meaning that the concentration of argon is of 50% (v/v). In other words, based on these measurements, it can be determined that the mixture generated is correct and contains 50% (v/v) Ar, 29% (v/v) N₂ and 21 % (v/v) O₂.

If the measured concentration far differs from 50% (v/v) Ar or 21 % (v/v) O₂, control unit 70 takes appropriate actions and shut off second solenoid valve 31 to stop the delivery of the therapeutic gas and solenoid valve 26b to stop the delivery of O₂ into second common line 50. Consequently, only air can fill the second mixing vessel 52.

The gas mixture obtained in the second mixing vessel 52 is recovered by a second exit line 53 and delivered by second outlet port 92 located at the end of the second exit line 53.

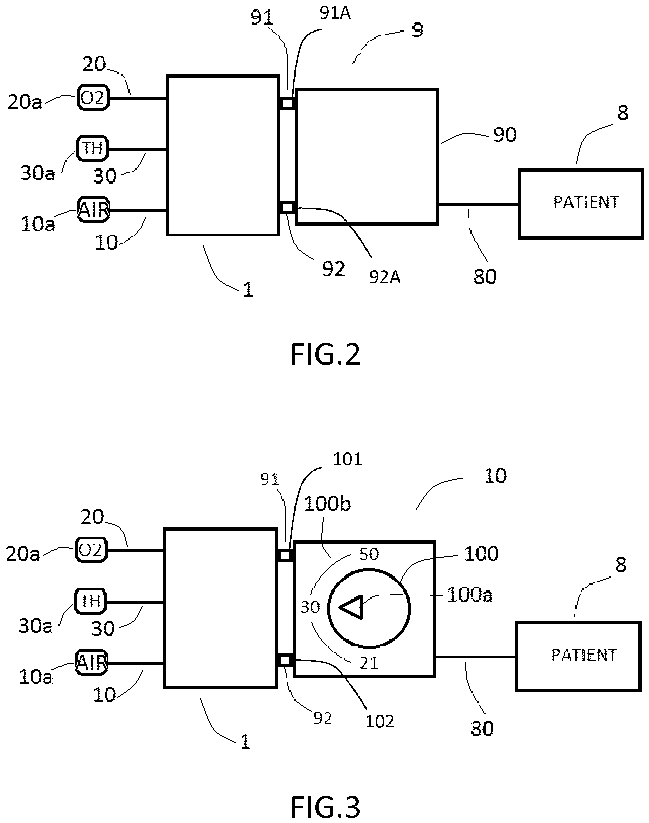

Figure 2 shows a mixer 1 according to the present invention, such as the mixer 1 of Figure 1, connected to the inlet orifices 91A, 92A of a mechanical ventilator 9 for feeding said ventilator 9 with a desired gas mixture and afterwards a patient 8 that is mechanically-ventilated. The mechanical ventilator 9 delivers a ventilation to the patient 8 via a breathing circuit 80, such as gas hose(s) or the like, fluidly connected to said ventilator 9.

In this embodiment, the first inlet 91A of ventilator 9 receives a mixture of 50% (v/v) Ar and 50% (v/v) O₂ at a pressure greater than 2 bars provided by the first outlet orifice 91 of the mixer 1, whereas the second inlet 92A of ventilator 9 receives a mixture of 50% (v/v) Ar, 21 % (v/v) O₂ and 29% (v/v) N₂ provided by the second outlet orifice 92 of the mixer 1.

Further, ventilator 9 is equipped with control means 90, such as a man-machine interface, for instance a keyboard, a touch screen or the like, allowing setting the O₂ concentration to be delivered to the patient, i.e. of between 21 and 50 vol. %.

With the mixer 1 of the present invention, various gas mixtures can be made and supplied to ventilator 9, for instance :
- For an O₂ concentration of 50 vol.%, only inlet 91A is used, i.e. fed with gas coming from the first mixing vessel 42 of the mixer 1. The gas mixture thus obtained contains 50% Ar and 50% O₂ (v/v%).
- For an O₂ concentration set at 21%, only inlet 92A is used and the gas stored in vessel 52 is fed to the ventilator 9, and afterwards delivered to the patient 8. The gas mixture thus obtained contains 50% Ar, 21% O₂ and 29% N₂. (v/v %)
- For an O₂ concentration set at 30%, both inlets 91A and 92A of ventilator 9 are used and the gas mixtures stored in both first and second mixing vessels 42, 52 are supplied to the ventilator 9. In this case, the mixture delivered to patient 8 is of 50%, Ar and 30% O₂ and 20% N₂ (v/v%).

Thanks to the mixer 1 of the present invention, the therapeutic gas (e.g. argon) can be precisely delivered to patient 8 regardless of the imprecisions of ventilator 9.

Further, whatever the set O₂ concentration, there is no need to flush the mixing vessels 42, 52 as the therapeutic gas concentration remains steady.

Furthermore, the mixer 1 of the present invention can be connected to any type of ventilator 9, e.g. neonatal, adults, transport ventilators.

For some patients, the use of medical ventilator as shown in Figure 2, is not compulsory, for instance when a patient 8 breathes spontaneously. However, adjusting the O₂ concentration between 21 and 50% (vol.%) can be necessary, while providing a steady therapeutic gas concentration at 50% (v/v) of Ar.

This can be done with the gas mixer 1 according to the present invention and a gas blender device as shown in Figure 3.

In this embodiment, the gas outlets 91, 92 of the gas mixer 1 are connected to the inlets 101, 102 of a blender device 10 comprising a rotatable dial 100, an indicator 100a, such as an arrow or any other marking, and O₂ concentration scale 100b arranged around the rotatable dial 100.

The position of the indicator 100a, relative to the O₂ concentration scale 100b, i.e. O₂ concentration range, determines the quantity of gases passing through inlets 101,102.

For instance, indicator 100a pointing to :
- the value "21" of the O₂ concentration scale 100b allows gas entering only into inlet 101,
- the value "50" allows gas entering only into inlet 102, and
- any value between "21" and "50" allows gas entering into both inlets 101, 102 of the blender device 10.

Consequently, a continuous flow is delivered to a patient 8, at the right therapeutic level (e.g. 50% (v/v) Ar), independently of the O₂ concentration set by dial 100.

Of course, according to another embodiment (not shown), blender device 10 can also be directly integrated into the housing of gas mixer 1.

Generallly speaking, an improved gas mixer according to the present invention can be used for providing ternary gas mixtures containing a therapeutically-effective gas(es), such as argon, at a concentration within tight tolerances, including when the oxygen concentration varies, and can be further fluidly connected to a medical ventilator or to a blender device for providing said ternary gas mixtures to said medical ventilator or to said blender device.

## Claims

1. Gas mixer (1) comprising:
- a first mixing vessel (42) and a second mixing vessel (52),
- a first line (22) for providing a first gas and a second line (32) for providing a second gas, the first and second lines (22, 32) being in fluid communication with the first mixing vessel (42) and with the second mixing vessel (52) for proving said first and second gases to said first and second mixing vessels (42, 52), and
- a third line (12) for providing a third gas, said third line (12) being in fluid communication with the second mixing vessel (52).

2. Gas mixer according to Claim 1, **characterized in that** the first line (22) is fluidly connected to the second mixing vessel (52) by a first additional line (261).

3. Gas mixer according to Claim 1, **characterized in that** the second line (32) is fluidly connected to the second mixing vessel (52) by a second additional line (361).

4. Gas mixer according to Claim 1 or 2, **characterized in that** the first additional line (261) comprises a flow control device (26b), preferably the flow control device (26b) is a solenoid valve.

5. Gas mixer according to Claim 1 to 4, **characterized in that**:
- the first and second lines (22, 32) are in fluid communication with the first mixing vessel (42) via a first common line section (40), and/or
- the first, second and third lines (22, 32, 12) are in fluid communication with the second mixing vessel (52) via a second common line section (50).

6. Gas mixer according to any one of the preceding Claims, **characterized in that**:
- the first line (22) comprises a first solenoid valve (21) and a first pressure regulator (23),
- the second line (32) comprises a second solenoid valve (31) and a second pressure regulator (33), and/or
- the third line (12) comprises a third solenoid valve (11) and a third pressure regulator (13).

7. Gas mixer according to any one of the preceding Claims, **characterized in that**:
- the first line (22) comprises a first calibrated orifice (25a),
- the second line (32) comprises a second calibrated orifice (35a), and/or
- the third line (12) comprises a third calibrated orifice (14a).

8. Gas mixer according to any one of the preceding Claims, **characterized in that**:
- the second additional line (361) comprises a first flow sensor (36b), and/or
- the second common line (50) comprises a second flow sensor (51b).

9. Gas mixer according to any one of the preceding Claims, **characterized in that** a first oxygen sensor (41) is arranged in first common line section (40), and/or a second oxygen sensor (51a) is arranged in second common line (50).

10. Gas mixer according to any one of the preceding Claims, **characterized in that** it further comprises a control unit (70) comprising a microprocessor.

11. Gas mixer according to any one of the preceding Claims, **characterized in that** the control unit (70) is electrically connected to :
- the first flow sensor (36b) of the second additional line (361),
- the second flow sensor (51b) of the second common line (50),
- the first oxygen sensor (41) of the first common line section (40), and/or
- the second oxygen sensor (51a) of the second common line (50).

12. Gas mixer according to any one of the preceding Claims, **characterized in that** the control unit (70) pilots :
- the first solenoid valve (21) and the first pressure regulator (23) of the first line (22),
- the second solenoid valve (31) and the second pressure regulator (33) of the second line (32), and/or
- the third solenoid valve (11) and the third pressure regulator (13) of the third line (12).

13. Gas mixer according to any one of the preceding Claims, **characterized in that**:
- the first line (22) is fed by a first gas source (20a) containing oxygen,
- the second line (32) is fed by a second gas source (30a) containing a therapeutically-effective gas, preferably argon, and
- the third line (12) is fed by a third gas source (10a) containing air.

14. Mechanical ventilation system comprising gas mixer (1) according to any one of the preceding Claims in fluid communication with a mechanical ventilator (9) for providing gas or gas mixtures to said mechanical ventilator (9).

15. Ventilation assembly comprising a gas mixer (1) according to any one of the Claims 1 to 14 in fluid communication with a blender device (10) for providing gas or gas mixtures to said blender device (10), especially an argon-containing gas mixture.

## Amended claims

### Amended claims in accordance with Rule 137(2) EPC.

1. Gas mixer (1) comprising:
- a first mixing vessel (42) and a second mixing vessel (52),
- a first line (22) for providing a first gas and a second line (32) for providing a second gas, the first and second lines (22, 32) being in fluid communication with the first mixing vessel (42) and with the second mixing vessel (52) for proving said first and second gases to said first and second mixing vessels (42, 52), and
- a third line (12) for providing a third gas, said third line (12) being in fluid communication with the second mixing vessel (52),
**characterized in that**:
- the first line (22) comprises a first solenoid valve (21) and a first pressure regulator (23),
- the second line (32) comprises a second solenoid valve (31) and a second pressure regulator (33), and
- the third line (12) comprises a third solenoid valve (11) and a third pressure regulator (13),
and further the gas mixer (1) comprises a control unit (70) for piloting :
- the first solenoid valve (21) and the first pressure regulator (23) of the first line (22),
- the second solenoid valve (31) and the second pressure regulator (33) of the second line (32), and
- the third solenoid valve (11) and the third pressure regulator (13) of the third line (12).

2. Gas mixer according to Claim 1, **characterized in that** the first line (22) is fluidly connected to the second mixing vessel (52) by a first additional line (261).

3. Gas mixer according to Claim 1, **characterized in that** the second line (32) is fluidly connected to the second mixing vessel (52) by a second additional line (361).

4. Gas mixer according to Claim 1 or 2, **characterized in that** the first additional line (261) comprises a flow control device (26b), preferably the flow control device (26b) is a solenoid valve.

5. Gas mixer according to Claim 1 to 4, **characterized in that**:
- the first and second lines (22, 32) are in fluid communication with the first mixing vessel (42) via a first common line section (40), and/or
- the first, second and third lines (22, 32,12) are in fluid communication with the second mixing vessel (52) via a second common line section (50).

6. Gas mixer according to any one of the preceding Claims, **characterized in that**:
- the first line (22) comprises a first calibrated orifice (25a),
- the second line (32) comprises a second calibrated orifice (35a), and/or
- the third line (12) comprises a third calibrated orifice (14a).

7. Gas mixer according to any one of the preceding Claims, **characterized in that**:
- the second additional line (361) comprises a first flow sensor (36b), and/or
- the second common line (50) comprises a second flow sensor (51b).

8. Gas mixer according to any one of the preceding Claims, **characterized in that** a first oxygen sensor (41) is arranged in first common line section (40), and/or a second oxygen sensor (51a) is arranged in second common line (50).

9. Gas mixer according to any one of the preceding Claims, **characterized in that** the control unit (70) is electrically connected to :
- the first flow sensor (36b) of the second additional line (361),
- the second flow sensor (51b) of the second common line (50),
- the first oxygen sensor (41) of the first common line section (40), and/or
- the second oxygen sensor (51a) of the second common line (50).

10. Gas mixer according to any one of the preceding Claims, **characterized in that**:
- the first line (22) is fed by a first gas source (20a) containing oxygen,
- the second line (32) is fed by a second gas source (30a) containing a therapeutically-effective gas, preferably argon, and
- the third line (12) is fed by a third gas source (10a) containing air.

11. Mechanical ventilation system comprising gas mixer (1) according to any one of the preceding Claims in fluid communication with a mechanical ventilator (9) for providing gas or gas mixtures to said mechanical ventilator (9).

12. Ventilation assembly comprising a gas mixer (1) according to any one of the Claims 1 to 10 in fluid communication with a blender device (10) for providing gas or gas mixtures to said blender device (10), especially an argon-containing gas mixture.
